**Europäisches Patentamt**

**European Patent Office**

**Office européen des brevets**

(19)

(11) Veröffentlichungsnummer : **0 084 089**
**B1**

(12) **EUROPÄISCHE PATENTSCHRIFT**

(45) Veröffentlichungstag der Patentschrift :
**15.05.85**

(51) Int. Cl.⁴ : **A 61 F   2/34**

(21) Anmeldenummer : **82110233.2**

(22) Anmeldetag : **06.11.82**

(54) **Im Becken verankerbare Gelenkpfanne.**

(30) Priorität : **12.01.82 CH 147/82**

(43) Veröffentlichungstag der Anmeldung :
**27.07.83 Patentblatt 83/30**

(45) Bekanntmachung des Hinweises auf die Patenterteilung : **15.05.85 Patentblatt 85/20**

(84) Benannte Vertragsstaaten :
**AT BE DE FR GB IT NL**

(56) Entgegenhaltungen :
**DE-A- 2 039 731**
**US-A- 4 123 806**
**US-A- 4 224 698**

(73) Patentinhaber : **GEBRÜDER SULZER AKTIENGE-
SELLSCHAFT**
**Zürcherstrasse 9**
**CH-8401 Winterthur (CH)**

**Protek AG**
**Stadtbachstrasse 64**
**CH-3001 Bern (CH)**

(72) Erfinder : **Niederer, Gino, Dipl.-Ing. ETH**
**Reichenbachstrasse 6**
**CH-3052 Zollikofen (CH)**
Erfinder : **Frey, Otto**
**Walrütistrasse 56**
**CH-8400 Winterthur (CH)**

(74) Vertreter : **Dipl.-Ing. H. Marsch Dipl.-Ing. K. Sparing**
**Dipl.-Phys. Dr. W.H. Röhl Patentanwälte**
**Rethelstrasse 123**
**D-4000 Düsseldorf (DE)**

## Beschreibung

Die Erfindung betrifft eine im Becken verankerbare Gelenkpfanne aus nicht metallischem Werkstoff zur Aufnahme eines kugeligen Gelenkkopfes einer Hüftgelenkprothese in einer halbkugelförmigen Pfannenschale, wobei der im Becken zu verankernde Aussenmantel der Gelenkpfanne mindestens teilweise eine rotationsfläche ist, und wobei ferner im Aussenmantel nahe dem Offenen Rand der Pfannenschale ein metallischer Kontrastring eingelegt ist.

Künstliche Hüftgelenkpfannen, in denen der Kugelkopf einer Hüftgelenkprothese gelagert ist, bestehen häufig aus Kunststoff, vor allem aus Polyäthylen, oder aus einem keramischen Werkstoff, beispielsweise Aluminiumoxid ; um ihre Sichtbarkeit im Röntgenbild zu verbessern, ist in den Aussenmantel derartiger Pfannen im allgemeinen nahe des offenen Randes der Pfannenschale ein Drahtring aus Metall als Kontrasting eingelegt (US-A-4 123 806).

Werden durch Vergleiche von Röntgenaufnahmen unmittelbar nach der Implantation und nach einem oder mehreren Jahren Relativ-Verschiebungen des Kugelkopfes in der Gelenkpfanne bzw. relativ zu dem Kontrastring festgestellt, so können diese Lageänderungen zwei Ursachen haben. Zum einen kann ein Kaltfluss, d. h. eine irreversible plastische Verformung, innerhalb des Pfannenlagers erfolgt sein ; zum anderen kann diese Relativ-Verschiebung durch Abrieb verursacht sein. Selbstverständlich kann eine solche Lageänderung auch teils durch Kaltfluss und teils durch Abrieb entstehen.

Da ein Kaltfluss des Pfannenmaterials im allgemeinen keine oder nur geringe physiologische Wirkungen entfaltet, während ein über das normale Mass — von etwa 0,1 mm pro Jahr — hinausgehender Abrieb auf die Dauer bedenklich ist, ist es wünschenswert, die Ursache der im Röntgenbild auftretenden relativen Lageänderungen des Kugelkopfes in der Gelenkpfanne erkennen zu können.

Aufgabe der Erfindung ist es daher, eine künstliche Gelenkpfanne der eingangs genannten Art zu schaffen, bei der aus Röntgenbildern des implantierten Gelenkes ermittelt werden kann, ob Aenderungen der relativen Lage des Kugelkopfes der Gelenkprothese in der Gelenkpfanne vor allem durch Kaltfluss oder in erster Linie durch Abrieb verursacht sind. Erfindungsgemäss wird diese Aufgabe gelöst durch einen, einen bestimmten Durchmesser aufweisenden metallischen Messring, der konzentrisch mit der Rotationsachse des Aussenmantels und parallel sowie in vorgegebenem vertikalen Abstand zur Fläche des Kontrastringes im Aussenmantel fixiert ist.

Neben der Lösung des geschilderten Problems, das noch anhand eines Ausführungsbeispiels ausführlich erläutert wird, bringt die erfindungsgemässe Massnahme noch den weiteren Vorteil, dass mit Hilfe der beiden Metallringe die Lage der implantierten Gelenkpfanne im Beckenknochen kontrolliert werden kann ; insbesondere sind dabei die Inklination der Pfanne — der Inklinationswinkel zwischen Pfannen- und Körperachse soll möglichst etwa 40° bis 45° betragen — und ihre Anteversion, deren Winkel im Optimum 12° betragen soll, festzustellen. Die Inklination ist bekanntlich die Neigung der Gelenkpfanne, d. h. beispielsweise ihrer Randebene, gegen die Horizontale bzw. die Neigung der Pfannenachse gegen die vertikale Körperachse, während die Anteversion eine Drehung der Pfanne um die in der Frontalebene des Körpers gelegene Durchmesserachse ihrer Randebene in sagittaler Richtung nach vorne ist.

Um den Abstand zwischen beiden Metallringen möglichst gross zu machen — und damit die relative Messgenauigkeit bei der Abstandsmessung zu erhöhen — ist es vorteilhaft, wenn der Messring im Scheitelbereich des Aussenmantels angeordnet ist. Weiterhin ist es für die Bestimmung des Sollmittelpunktes des Gelenkkugelkopfes zweckmässig, wenn der Kontrastring in der Durchmesserebene der halbkugelförmigen Pfannenschale angeordnet ist.

Als Materialien für die Herstellung der beiden Ringe können alle in der Implantattechnik üblichen Metalle bzw. Metall-Legierungen verwendet werden ; die Gelenkpfannen selbst sind in erster Linie aus Kunststoff, insbesondere aus Polyäthylen der spezifikationen HDPE und UHMW, gefertigt.

Im folgenden wird die Erfindung anhand von Ausführungsbeispielen im Zusammenhang mit der Zeichnung näher erläutert.

Figur 1 zeigt im Schnitt schematisch eine erfindungsgemäss mit zwei Ringen ausgestattete Gelenkpfanne ;

Figur 2 gibt die Ansicht wieder, in der eine implantierte Prothese in einem Röntgenbild mit Durchstrahlung in sagittaler Richtung erscheint.

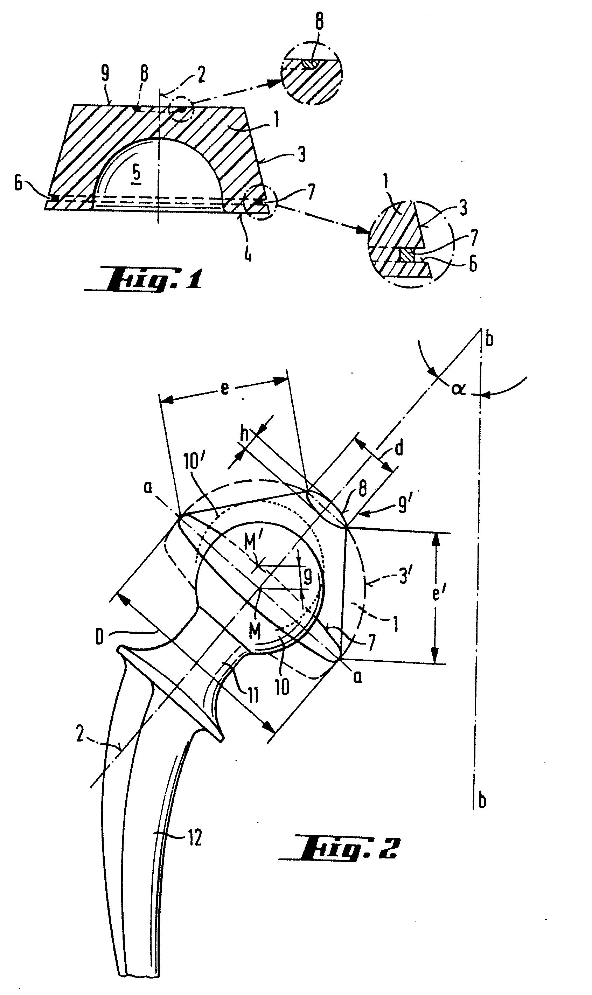

Der Pfannenkörper 1 (Fig. 1) ist ein kegelstumpfförmiger Rotationskörper, dessen Rotationsachse mit 2 bezeichnet ist. Konzentrisch mit seinem Aussenmantel 3 erstreckt sich von seiner Basis 4 aus die eigentliche Pfannenschale 5 als Hohlhalbkugel in den Pfannenkörper 1 hinein.

Nahe dem basisseitigen Rand ist in den Aussenmantel 3 eine Nut 6 eingeschnitten, in die ein metallischer Kontrastring 7 eingelegt ist.

Ein weiterer Metallring ist erfindungsgemäss als Messring 8 in die obere Stirnfläche 9 des Kegelstumpfes eingelassen ; er besitzt einen bekannten definierten Durchmesser und liegt konzentrisch zum Aussenmantel 3, d. h. die Rotationsachse 2 bildet auch seine Achse ; seine Ringfläche ist parallel zur Ringfläche des Kontrastringes 7.

Bei Gelenkpfannen mit halbkugelförmigem Aussenmantel 3', wie sie in Fig. 2 gestrichelt angedeutet ist, ist der Messring 8 mit Vorteil im Scheitelbereich 9' der Halbkugel 3' angeordnet. Sein Abstand vom Kontrastring 7 und damit sein Umfang sind dabei beliebig wählbar ; jedoch sollte sein Abstand vom Kontrastring 7 so gross sein, dass der Messring 8 bei einer Röntgenaufnahme, wie sie der fig. 2 zugrundeliegt, nicht mehr in den Schattenbereich des Kugelkopfes 10 fällt.

In Fig. 2 ist der Kugelkopf 10, der über einen Prothesenhals 11 auf einem Schaft 12 sitzt, mit ausgezogenen Linien in seiner Soll-Lage dargestellt, in der er sich unmittelbar nach der Implantation befindet.

Die gestrichelt angedeutete Gelenkpfanne hat aufgrund ihrer Anteversion im körper — und damit im Röntgenbild der erwähnten Durchstrahlrichtung — eine Lage, in der der Kontrastring 7 und der Messring 8 als schmale Ellipsen erscheinen.

Zur Ermittlung des Inklinationswinkels bestimmt man zunächst mit Hilfe des grössten auf dem Röntgenbild vorhandenen Durchmessers D des Kontrastringes 7 die Richtung der grossen Ellipsenachse aa dieses Ringes ; die Senkrechte auf diese Achse aa bildet mit der Körperachse bb den Inklinationswinkel α.

Der Anteversionswinkel lässt sich durch Ausmessen der beiden Achsen d und h des im Röntgenbild als Ellipse erscheinenden Messrings 8 ermitteln ; je grösser dabei h relativ zu d ist, umso grösser ist die Anteversion der Gelenkpfanne.

In punktierten Linien ist in Fig. 2 eine als Beispiel angenommene Lage des Umrisses 10' des Kugelkopfes 10 bei einer späteren Aufnahme, die längere Zeit nach der Implantation gemacht worden ist, gezeigt. Die im Zeitraum zwischen beiden Aufnahmen erfolgte Lageänderung g des Kugelmittelpunktes M' gegenüber dem ursprünglichen oder Sollmittelpunktes M lässt sich auf folgende Weise bestimmen :

Man misst die grossen Halbachsen D bzw. d der beiden « Ellipsen » der Ringe 7 und 8 und halbiert sie ; die kürzeste, d. h. die senkrechte, Verbindung der beiden Achsenmittelpunkte legt die Rotationsachse 2 für die Gelenkpfanne 1 fest. Ihr Schnittpunkt mit der grossen Halbachse aa des Kontrastrings 7 hat einen definierten, je nach Pfannentyp verschiedenen Abstand zum Sollmittelpunkt M des Kugelkopfes 10 unmittelbar nach der Implantation ; bei dem gezeigten Ausführungsbeispiel fallen Schnittpunkt und Sollmittelpunkt des Kugelkopfes 10 zusammen, ist also dieser Abstand = O. Den Mittelpunkt M' des Kugelkopfes 10' in der veränderten Lage erhält man aus der grössten Abmessung des Kugelkopfbildes, die den Durchmesser des Kugelkopfes 10' ergibt, auf dem sich M' in der Mitte befindet. Der kürzeste Abstand M-M' ist die gesuchte Lageänderung g.

Diese in Fig. 2 stark übertrieben dargestellte Lageänderung g des Kugelkopfes 10 kann durch Kaltfliessen und/oder durch Abrieb verursacht sein.

Ein Kaltfluss ist dabei aus dem Röntgenbild zu erkennen, wenn man den Abstand e bzw. e' zwischen den beiden grossen Achsen D und d der beiden « Ellipsen » des Kontrastrings 7 und des Messrings 8 auf der linken Seite und auf der rechten Seite miteinander vergleicht. Sind beide Abstände e bzw. e' links und rechts gleich, so liegt kein Kaltfluss des Pfannenmaterials vor, während diese Abstände links und rechts verschieden sind, sobald ein Kaltfliessen des Materials stattgefunden hat.

Somit lässt sich aufgrund der Erfindung bei einer festgestellten Lageveränderung g bestimmen, ob diese allein durch Abrieb verursacht oder zumindest teilweise durch Kaltfluss des Materials hervorgerunfen ist.

**Patentansprüche**

1. Im Becken verankerbare Gelenkpfanne (1) aus nicht metallischem Werkstoff zur Aufnahme eines kugeligen Gelenkkopfes (10, 10') einer Hüftgelenkprothese in einer halbkugelförmigen Pfannenschale (5), wobei der im Becken zu verankernde Aussenmantel (3, 3') der Gelenkpfanne (1) mindestens teilweise eine Rotationsfläche ist, und wobei ferner im Aussenmantel (3, 3') nahe dem Offenen Rand der Pfannenschale (5) ein metallischer Kontrastring (7) eingelegt ist, gekennzeichnet durch einen, einen bestimmten Durchmesser aufweisenden metallischen Messring (8), der konzentrisch mit der Rotationsachse (2) des Aussenmantels (3, 3') und parallel sowie in vorgegebenem, vertikalen Abstand zur Fläche des Kontrastrings (7) im Aussenmantel (3, 3') fixiert ist.

2. Gelenkpfanne nach Anspruch 1, dadurch gekennzeichnet, dass der Messring (8) im Scheitelbereich (9') des Aussenmantels (3') angeordnet ist.

3. Gelenkpfanne nach Anspruch 1 oder 2, dadurch gekennzeichnet, dass der Kontrastring (7) in der Durchmesserebene der halbkugelförmigen Pfannenschale (5) angeordnet ist.

**Claims**

1. A non-metallic joint socket (1) which can be anchored in the pelvis and which in adapted to receive a spherical joint head (10, 10') of a hip joint prosthesis in a hemispherical socket shell (5), the socket outside surface (3, 3') for anchorage in the pelvis being at least to some extent a surface of rotation, a metal contrast ring (7) being disposed in the said outer surface (3, 3') near the open edge of the shell (5), characterised by a metal measuring ring (8) which has a predetermined diameter and which is located concentrically of the rotational axis (2) of the said outside surface (3, 3') and parallel and at a predetermined vertical distance from the contrast ring surface in the said outside surface (3, 3').

2. A joint socket according to claim 1, characterised in that the measuring ring (8) is disposed in the apex zone (9') of the said outside surface (3').

3. A joint socket according to claim 1 or 2, characterised in that the contrast ring (7) is disposed in the diametric plane of the hemispherical socket shell (5).

**Revendications**

1. Cuvette d'articulation (1) en un matériau non métallique, pouvant être ancrée dans le bassin pour recevoir la tête sphérique d'articulation (10, 10') d'une prothèse coxofémorale dans une calotte hémisphérique (5) de la cuvette, l'enveloppe externe (3, 3') de la cuvette d'articulation (1), devant être ancrée dans le bassin, constituant au moins partiellement une surface de rotation, et une bague métallique de contraste (7) étant en outre incorporée dans l'enveloppe externe (3, 3') à proximité du bord ouvert de la calotte (5) de la cuvette, caractérisée par une bague métallique de mesure (8) qui, présentant un diamètre déterminé, est assujettie dans l'enveloppe externe (3, 3') concentriquement à l'axe de rotation (2) de cette enveloppe externe (3, 3') et parallèlement à la surface de la bague de contraste (7), ainsi qu'à une distance verticale prédéterminée de cette surface.

2. Cuvette d'articulation selon la revendication 1, caractérisée par le fait que la bague de mesure (8) se trouve dans la région du sommet (9') de l'enveloppe externe (3').

3. Cuvette d'articulation selon la revendication 1 ou 2, caractérisée par le fait que la bague de contraste (7) est disposée dans le plan diamétral de la calotte hémisphérique (5) de la cuvette.

*Fig. 1*

*Fig. 2*

1